Europäisches Patentamt

European Patent Office

Office européen des brevets

(19)

(11) Publication number: **0 208 090**
**B1**

(12) **EUROPEAN PATENT SPECIFICATION**

(45) Date of publication of the patent specification: 28.06.89

(51) Int. Cl.⁴: **A61M 1/14**

(21) Application number: 86106540.7

(22) Date of filing: 14.05.86

(54) An apparatus system for the control of a medical treatment, e.g. dialysis.

(30) Priority: 04.06.85 SE 8502758

(43) Date of publication of application:
14.01.87 Bulletin 87/3

(45) Publication of the grant of the patent:
28.06.89 Bulletin 89/26

(84) Designated Contracting States:
BE CH DE FR GB IT LI NL

(56) References cited:
CH-A- 618 881
DE-B- 2 200 481
FR-A- 2 502 959
US-A- 3 441 136
US-A- 3 738 382
US-A- 3 878 095
US-A- 3 882 020
US-A- 4 122 010
US-A- 4 137 168
US-A- 4 153 554

(73) Proprietor: **Gambro AB, Fack, S-220 10 Lund(SE)**

(72) Inventor: **Petersen, Preben Allan, Kamrersvägen 46,
S-237 00 Bjärred(SE)**
Inventor: **Sternby, Jan Peter, Sparsnögatan 45,
S-222 52 Lund(SE)**

(74) Representative: **Boberg, Nils Gunnar Erik, Gambro AB
Patent Department Box 10101, S-220 10 Lund(SE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

### TECHNICAL FIELD

The present invention relates to an apparatus for the control of medical treatment as defined in the preamble of claim 1 and known from US-A 4 122 010.

The invention primarily relates to a system for the control of haemodialysis, that is to say for the purifying of the blood of patients with diminished renal function or wholly lacking such function. It will be obvious, to those versed in the art that the system in accordance with the invention also may be used for the control of other medical treatments, such as peritoneal dialysis, haemofiltration, plasmaferes etc., that is to say systems comprising means for the tempering of a treatment fluid.

### BACKGROUND ART

More particularly the invention relates to a further development of the system which is marketed by the Gambro Group, to which the applicant belongs, under the description "Gambro AK-10".

Various details of this system form the subject of, for example the following US Patents: 4 122 010, 4 158 034, 4 293 409, 4 194 974 and 4 191 359, these descriptions being included hereby in the present description. Reference is made also to the further development according to European patent application EP 84.112914.1 and the invention according to the British patent 2 003 274 and the further development of this according to the European patent application published under number EP 0 106 940, and these too are included in the present description. Reference is made, furthermore, to EP 0 022 922 which describes in more detail the preparation of dialysis fluid from two concentrates, a preparation which is only touched upon in the following description of the present invention.

Finally, reference is made to US patent 3 738 382 which describes a competing system for the preparation of dialysis fluid wherein the sterilization is achieved with the help of a heating element normally included in the system, at the same time as the heated sterilizing agent is made to recirculate through the system in its entirety. The said system is subject to the disadvantage, among other things, that the sterilizing fluid is caused to recirculate through less contaminated as well as more contaminated parts of the system. The result can be that impurities may be passed from the more contaminated parts to the less contaminated parts. Furthermore, the known system is designed to operate at a pressure above atmospheric which is adapted to a very special procedure for the preparation of dialysis fluid, as a result of which its applicability is limited.

### DISCLOSURE OF INVENTION

In order to avoid these disadvantages, the invention proposes an apparatus as claimed in Claim 1. The advantage gained thanks to this design is that heating devices normally included in the system suffice, without overdimensioning, both for compensation of heat losses in the recirculating system and for the heating of a small amount of new fluid supplied continuously. At the same time an amount of fluid equal to that newly supplied may be allowed to flow through more contaminated parts of the system directly to an outlet.

Preferably the said recirculation is arranged so as to take place with the help of a special return line from a point further on in the system to a point at the start of the system. Appropriately this special return line is adapted so that the recirculation takes place from the said bypass arrangement, that is to say within the part of the system which normally contains pure treatment fluid not affected by the treatment arrangement. Thereby it also ensured that no recirculation is occasioned in the part of the system through which flows normally treatment fluid which has been acted on and thus may also have been contaminated by the treating arrangement.

If it is merely intended to procure disinfection, the normal heating device of the system may be adapted so as to raise the temperature in a fluid heating vessel to substantially 90°C at the same time as the recirculation selected is such that the temperature in the recirculation circuit does not fall far short of 90°C. At the same time the temperature in the system, after the point of recirculation, may be allowed gradually to drop, e.g. down to the order of magnitude of 80°C.

Preferably the disinfection and/or the sterilization are adapted so as to take place at substantially normal atmospheric pressure. If it is desired to assure effective sterilization it may become necessary, though, either to increase the pressure and the temperature or supplement it by chemical means.

In practice it has proved appropriate to allow the disinfection and/or the sterilization to take place with substantially maximum flow in the recirculation circuit, whilst the flow in the rest of the system is kept at a fraction of this, e.g. in the order of magnitude of one fifth.

Any constrictions included in the recirculation circuit are arranged appropriately to be opened or wholly or partly bridged over during disinfection and/or sterilization so as to minimize flow resistance.

### BRIEF DESCRIPTION OF DRAWINGS

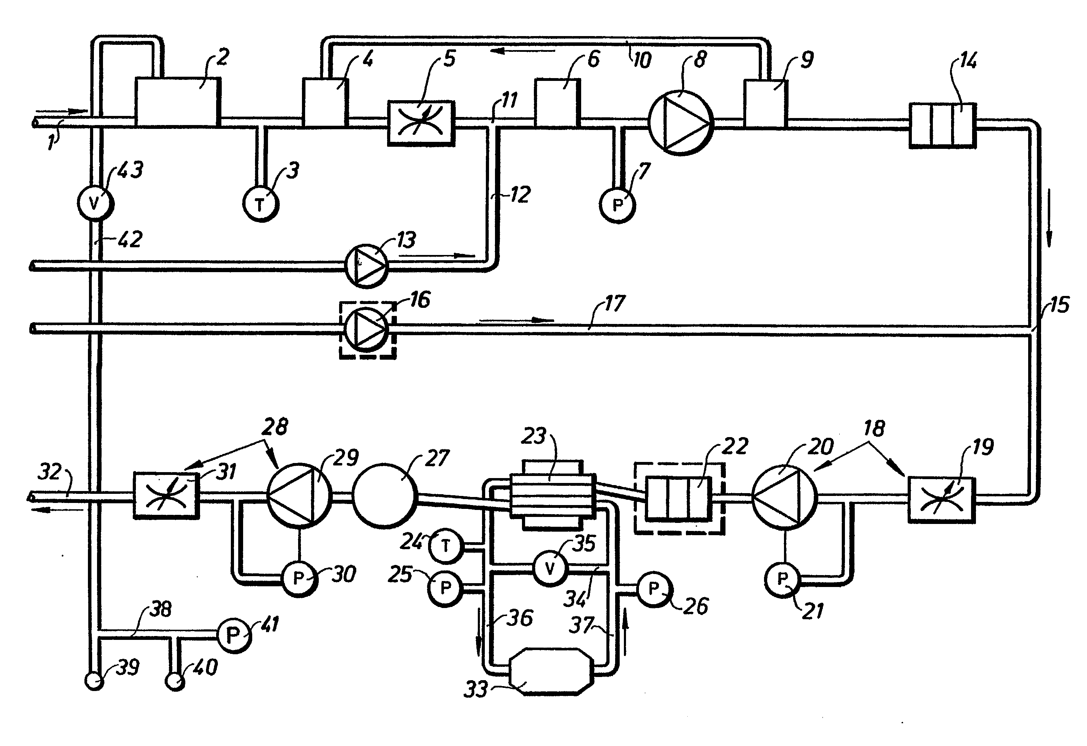

The invention is described in more detail in the following with reference to the attached drawing which, by way of example, shows the most important components of a system in accordance with the invention.

### BEST MODE OF CARRYING OUT THE INVENTION

In the system shown as an example in the attached drawing the water is fed via an inlet 1 to a heating device 2, where it is heated. The temperature is then measured in a temperature measuring device 3 before the water is conducted via a return vessel 4 to a constriction 5 and further via a bubble expansion tank 6, a pressure gauge 7 and a pump 8 to a vent tank 9. From the vent tank 9 a return duct 10 leads back to the return vessel 4 for the recycling of any air or other gases separated together with a small part of the fluid. A recycling would be possible instead to the heating vessel 2, but this would then require to be made from a more resistant material, since dialysis concentrate is being supplied to the point 11 via the duct 12 with the help of the pump 13. This part of the system corresponds in substantial parts to the system which is described, for example, in the US patents 4 158 034 and 4 293 409. The function of the expansion vessel 6 is described in detail in the European patent application 84.112914.1. From the vent tank 9 the fluid is passed further via a conductivity measuring cell 14 to another mixing point 15 where further concentrate is supplied possibly with the help of a pump 16 and a duct 17. This is done on the assumption that it is intended to work with so-called two-component-based dialysis concentrate, e.g. of the type which is described in EP 0 022 922.

From the mixing point 15 the dialysis fluid is passed through a first constant flow arrangement 18 consisting of a constriction 19, a pump 20 and a pressure gauge 21. Before the constriction device 19 the pressure is substantially at atmospheric pressure as the duct is connected without any major resistance to the vent tank 9. Should it be found in practice that the pressure varies owing to the pump 16, the conductivity meter 14 and the mixing point 15 can be moved to a position before the vent tank 9.

The pressure measured by the pressure gauge 21 is used for the control of the pump 20, so that a desired constant flow is obtained. After the pump 20 the flow is passed through a conductivity meter 22 and an ultrafiltration monitor 23 via a temperature measuring instrument 24 and a pressure gauge 25 to the dialyzer. From the dialyzer the flow is conducted via a pressure gauge 26, the said ultrafiltration monitor 23 and a blood detector 27 to a further constant flow arrangement 28 consisting of a pump 29, a pressure gauge 30 and a constriction device 31. Finally the dialysate is passed to an outlet 32. It is appropriate for the constant flow arrangement 28 to be identical with the arrangement 18. In the constriction 31, though, the pressure drop obtained is from a pressure above atmospheric to the pressure prevailing at the outlet which ought to be kept constant and, for example, may be equal to atmospheric pressure.

The design and function of the ultrafiltration monitor are described in greater detail in the abovementioned publications GB 2 003 274 and EP 0 106 940.

Numeral 33 designates the dialyzer which can be connected to the system in accordance with the invention, on the blood side of which the patient is connected up. The latter connection, however, is not shown in the drawing.

The dialyzer 33 can be bridged over in two ways. It may be done either with the help of the by-pass duct 34 with the valve 35 which is opened if, for example, the temperature or the conductivity exceed, or fall short of, certain limit values. At the same time the flow to the dialyzer 33 is stopped with the help of a valve not shown in the drawing.

Alternatively the bridging may be done in that the dialyzer connections 36 and 37 are coupled to a by-pass duct 38 via the couplings 39 and 40. This bridging arrangement is designed appropriately in the main according to US patent 4 122 010 with a pressure monitoring device 41 which checks whether a suction pressure exists in the duct 38. In the present case, though, a pressure above atmospheric is detected instead. If this is the case, and only then, sterilization may take place.

In the example shown a return duct 42 with a valve 43 runs back from the bridging arrangement 38 to the heating vessel 2. Through this duct 42 recirculates a part of the flow, when the system is to be disinfected or sterilized. The remainder of the flow is passed instead from the duct 36, which is connected to the coupling 39 via the duct 38 and the coupling 40 to the duct 37 and from there through the ultrafiltration monitor 23 and further to the outlet 32. A small amount, however, is conducted directly from the duct 36 via the duct 34 with the valve 35 directly to the duct 37 for disinfection and/or sterilization of this bridging arrangement.

Naturally the invention is not limited merely to the embodiment described above, but can be varied within the scope of the following claims. For example, the diverse components included in the system can be varied within wide limits without the scope of the invention thereby being exceeded. Compare, for example the publications cited above and the patent applications 85.02756-3 and 85.02757-1 with our references GA 139 and GA 140 submitted at the same time.

## Claims

1. An apparatus for the control of a medical treatment, e.g. dialysis, using a tempered fluid, comprising means (2, 3 and 18, 28 respectively) for the control of the temperature and the flow of this fluid, means (36, 37) for the connection of the system to a treating arrangement, e.g. a dialyzer (33), and means including a heating device (2) for disinfection and/or sterilization with the help of a fluid heated to a higher temperature than normal treatment temperature, this fluid being made to flow along the same path as the treatment fluid, except for being conducted past the said treating arrangement (33) through a so-called by-pass arrangement (38–41 and/or 34, 35), characterized by means (36, 42, 43) for recirculating a part of the fluid heated to the said higher temperature for repeated passage of the heating device (2), while the rest of the fluid is passed to an outlet (32) of the apparatus, and in that the said recirculation is arranged so as to take place with the help of a special return line (42) from a point further on in the system to a point at the start of the system.

2. An apparatus in accordance with claim 1, characterized in that the recirculation is arranged to take place from the said by-pass arrangement (38–41), that is to say within the part of the system which normally contains a treatment fluid not affected by the treating arrangement (33).

3. An apparatus in accordance with claim 2, characterized in that no recirculation is occasioned in the part of the apparatus through which flows normally treatment fluid which has been acted on, and thus may also have been contaminated, by the treating arrangement (33).

4. An apparatus in accordance with anyone of the preceding claims, characterized in that the normal heating device (2) of the system in the case of disinfection is adapted to raise the temperature in a fluid heating vessel to substantially 90°C and that such a recirculation is selected that the temperature in the recirculation circuit does not fall far short of 90°C, whilst the temperature in the system after the point of recirculation is allowed gradually to drop, e.g. down to the order of magniture of 80°C.

5. An apparatus in accordance with anyone of the preceding claims, characterized in that the disinfection and/or the sterilization are adapted so as to take place at substantially normal atmospheric pressure.

6. An apparatus in accordance with anyone of the preceding claims, characterized in that the disinfection and/or the sterilization are adapted so as to take place with substantially maximum flow in the recirculation circuit, whilst the flow in the rest of the system is adapted so as to be kept to a fraction of this, e.g. in the order of magnitude of one fifth.

7. An apparatus in accordance with anyone of the preceding claims, characterized in that any constrictions included in the recirculation circuit are arranged to be opened during disinfection and/or sterilization and/or are wholly or partly bridged over so as to minimize flow resistance.

**Revendications**

1. Appareil pour la commande d'un traitement médical, par exemple une dialyse, utilisant un fluide en température, qui comprend des moyens (2, 3 et 18, 28 respectivement) pour la commande de la température et du débit de ce fluide, des moyens (36, 37) pour le raccordement du système à un dispositif de traitement, par exemple un dialyseur (33), et des moyens comportant un dispositif de chauffage (2) pour la désinfection et/ou la stérilisation à l'aide d'un fluide chauffé à une température supérieure à la température normale de traitement, ce fluide étant amené à emprunter le même chemin que le fluide de traitement, sauf en ce qu'il est conduit au-delà dudit dispositif de traitement (33), par un dispositif dit de dérivation (38–41) et/ou 34, 35), caractérisé par des moyens (36, 42, 43) de recirculation d'une partie du fluide chauffé à ladite température plus élevée, pour un passage répété dans le dispositif de chauffage (2), tandis que le reste du fluide est envoyé à une sortie (32) de l'appareil, et en ce que ladite recirculation est prévue de manière à s'effectuer à l'aide d'une ligne de retour spéciale (42) à partir d'un point plus éloigné dans le système jusqu'à un point situé au début du système.

2. Appareil suivant la revendication 1, caractérisé en ce que la recirculation est prévue de manière à s'effectuer à partir dudit dispositif de dérivation (38–41), c'est-à-dire dans la partie du système qui contient normalement un fluide de traitement non affecté par le dispositif de traitement (33).

3. Appareil suivant la revendication 2, caractérisé en ce qu'aucune recirculation n'est effectuée dans la partie de l'appareil à travers laquelle circule normalement le fluide de traitement qui a servi et peut donc également avoir été contaminé par le dispositif de traitement (33).

4. Appareil suivant l'une quelconque des revendications précédentes, caractérisé en ce que le dispositif de chauffage normal (2) du système dans le cas de désinfection est prévu pour élever la température, dans une cuve de chauffage du fluide, sensiblement à 90°C et en ce qu'une telle recirculation est choisie de sorte que la température dans le circuit de recirculation atteint pratiquement 90°C, tandis que la température dans le système après le point de recirculation peut chuter progressivement, par exemple jusqu'à l'ordre de grandeur de 80°C.

5. Appareil suivant l'une quelconque des revendications précédentes, caractérisé en ce que la désinfection et/ou la stérilisation sont prévues pour être effectuées sensiblement à la pression atmosphérique normale.

6. Appareil suivant l'une quelconque des revendications précédentes, caractérisé en ce que la désinfection et/ou la stérilisation sont prévues pour être effectuées au débit sensiblement maximal dans le circuit de recirculation, tandis que le débit dans le reste du système et prévu pour être maintenu à une fraction du précédent, par exemple de l'ordre de grandeur d'une cinquième.

7. Appareil suivant l'une quelconque des revendications précédentes, caractérisé en ce que tous les étranglements inclus dans le circuit de recirculation sont prévus pour être ouverts pendant la désinfection et/ou la stérilisation et/ou sont contournés en totalité ou en partie de manière à minimiser la résistance à l'écoulement.

**Patentansprüche**

1. Apparatur für die Steuerung einer medizinischen Behandlung, z.B. einer Dialyse, unter Verwendung eines temperierten Fluids, mit Mitteln (2, 3 bzw. 18, 28) zum Steuern der Temperatur und des Flusses dieses Fluids, Mitteln (36, 37) für den Anschluß des Systems an eine Behandlungsanordnung, z.B. ein Dialysegerät (33), und Mitteln, einschließlich einer Heizvorrichtung (2), zur Desinfektion und/oder Sterilisation unter Zuhilfenahme eines Fluids, welches auf eine höhere als die normale Behandlungstemperatur aufgeheizt ist, wobei dieses Fluid dazu gebracht wird entlang desselben Weges wie das Behandlungsfluid zu fließen, außer daß es an der Bahandlungsanordnung (33) vorbei durch eine sogenannte Bypass-Anordnung (38–41 und/oder 34, 35) geleitet wird, gekennzeichnet durch Mittel (36, 42, 43) für die Rückführung eines Teiles des Fluids, welches auf die höhere Temperatur aufgeheizt ist, für einen wiederholten Durchtritt durch die Heizvorrichtung (2), während der Rest des Fluids durch einen Ausgang (32) der Apparatur geführt wird, und daß die Rückführung so angeordnet wird, daß sie mit Hilfe einer speziellen Rückführleitung (42) von einem Punkt weiter im Innern des Systems zu einem Punkt am Beginn des Systems stattfindet.

2. Apparatur nach Anspruch 1, dadurch gekennzeichnet, daß die Rückführung so angeordnet wird, daß sie von der Bypass-Anordnung aus (38–41) stattfindet, d.h. innerhalb des Teils des Systems, welches normalerweise ein Behandlungsfluid enthält, welches nicht von der Behandlungsanordnung (33) beeinflußt ist.

3. Apparatur nach Anspruch 2, dadurch gekennzeichnet, daß keine Rückführung stattfindet in dem Teil der Apparatur, durch welchen normalerweise Behandlungsfluid fließt, auf welches bereits durch die Behandlungsanordnung (33) eingewirkt wurde und welches daher auch schon verunreinigt sein kann.

4. Apparatur nach einem der vorstehenden Ansprüche, dadurch gekennzeichnet, daß die normale Heizvorrichtung (2) des Systems im Falle der Desinfektion so ausgelegt ist, daß sie die Temperatur in einem Fluidheizbehälter auf im wesentlichen 90°C erhöht und daß eine derartige Rückführung ausgewählt wird, daß die Temperatur in dem Rückführungskreislauf nicht weit unter knapp 90°C fällt, während zugelassen wird, daß die Temperatur in dem System hinter dem Rückführungspunkt allmählich fällt, z.B. auf die Größenordnung von 80°C.

5. Apparatur nach einem der vorstehenden Ansprüche, dadurch gekennzeichnet, daß die Desinfektion und/oder die Sterilisation so angepaßt werden, daß sie im wesentlichen bei normalem atmosphärischen Druck stattfinden.

6. Apparatur nach einem der vorstehenden Ansprüche, dadurch gekennzeichnet, daß die Desinfektion und/oder Sterilisation so ausgelegt sind, daß sie im wesentlichen mit maximalem Fluß in dem Rückführungskreis stattfinden, während der Fluß in dem übrigen System so ausgelegt ist, daß er bei einem Bruchteil von jenem gehalten wird, z.B. in der Größenordnung von einem Fünftel.

7. Apparatur nach einem der vorstehenden Ansprüche, dadurch gekennzeichnet, daß jegliche Einengungen, welche in dem Rückführungskreislauf enthalten sind, so angeordnet sind, daß sie während der Desinfektion und/oder Sterilisation geöffnet werden können und/oder ganz oder teilweise überbrückt werden, so daß der Flußwiderstand minimal gemacht wird.

EP 0 208 090 B1